# EUROPÄISCHE PATENTSCHRIFT

(11) **EP 3 069 576 B1**
(45) Veröffentlichungstag und Bekanntmachung des Hinweises auf die Patenterteilung: **27.09.2017**
(21) Anmeldenummer: 14809279.4
(22) Anmeldetag: 07.11.2014
(51) Int. Cl.: H05H 1/24, A61L 2/14

(54) **GERÄT ZUR BEHANDLUNG EINER FLÄCHE MIT EINEM PLASMA**
DEVICE FOR TREATING A SURFACE WITH A PLASMA
APPAREIL DE TRAITEMENT D'UNE SURFACE AU MOYEN D'UN PLASMA

(30) Priorität: 15.11.2013 DE 102013019058
(43) Veröffentlichungstag der Anmeldung: 21.09.2016
(73) Patentinhaber: Cinogy GmbH, 37115 Duderstadt (DE)
(72) Erfinder: WANDKE, Dirk, 37308 Heilbad Heiligenstadt (DE); TRUTWIG, Leonhard, 37115 Duderstadt (DE); HAHNL, Mirko, 37339 Berlingerode (DE); STORCK, Karl-Otto, 37115 Duderstadt (DE)
(74) Vertreter: Lins, Martina
(86) Internationale Anmeldenummer: PCT/DE2014/000578
(87) Internationale Veröffentlichungsnummer: WO 2015/070835

(56) Entgegenhaltungen:
- WO-A2-01/87166
- DE-A1-102011 105 713
- DE-A1-102012 103 470

## Beschreibung

Die Erfindung betrifft ein Gerät zur Behandlung einer Fläche mit einem dielektrisch behinderten Plasma gemäß Anspruch 1. Ein Gerät zur Behandlung einer Fläche mit einem dielektrisch behinderten Plasma ist durch DE 10 2007 030 915 A1 bekannt. Eine längliche Elektrode mit einem zylindrischen Querschnitt und einer abgerundeten Stirnseite ist dabei von einem entsprechend ausgebildeten Dielektrikum umgeben. Mit der Mantelfläche des Dielektrikums kann eine Fläche, beispielsweise ein Hautareal, behandelt werden. Eine gleichmäßige Behandlung einer größeren Fläche ist mit einer derartigen Vorrichtung nicht vorgesehen.

Durch DE 10 2009 060 627 ist für die Behandlung größerer Hautareale eine flächige Elektrodenanordnung vorgesehen, bei der eine flächige Elektrode durch ein flächiges Dielektrikum gegenüber einer zu behandelnden Fläche, insbesondere Hautfläche, abgeschirmt wird. Zur Anpassung an unregelmäßige Oberflächen ist sowohl die Elektrode als auch das Dielektrikum flexibel ausgebildet. Eine derartige flächige Elektrode kann auf die zu behandelnde Fläche aufgelegt werden, wobei das Dielektrikum strukturiert ausgebildet ist um zwischen Haut und Dielektrikum einen Luftzwischenraum zu befassen, in dem die Plasmaentladung stattfinden kann, wenn die zu behandelnde Fläche als Gegenelektrode verwendet wird. Nachteilig an dieser Anordnung ist die Großflächigkeit der Elektrodenanordnung, die vorzugsweise an einer stationären Einrichtung verwendbar ist und darüber hinaus die Behandlung von stark gekrümmten Flächen nur bedingt ermöglicht.

Durch DE 10 2012 103 470 A1 ist ein Gerät der eingangs erwähnten Art bekannt. Über eine kugelförmige Elektrode, die sich in einem Gehäuse frei drehen kann, soll eine Behandlung einer Oberfläche mit einem dielektrisch behinderten Plasma erfolgen. Die Einkopplung einer Wechsel-Hochspannung soll über eine Koppelelektrode kapazitiv erfolgen. Dies hat zur Folge, dass der Stromfluss zur kugelförmigen Elektrode bereits dielektrisch behindert ist. Wenn die Elektrode ohne eine dielektrische Abschirmung auf der zu behandelnden Oberfläche anliegt, entsteht somit um den punktförmigen Kontakt zwischen der Kugel und der zu behandelnden Oberfläche ein ringförmiger Bereich, in dem sich das dielektrisch behinderte Plasma ausbilden kann. Wenn allerdings gemäß einem anderen Ausführungsbeispiel die kugelförmige Elektrode mit einer dielektrischen Schicht umhüllt ist, kann sich mit einem vernünftigen Aufwand kein Plasma mehr an der Oberfläche ausbilden. Die Behandlung von leitfähigen Oberflächen, bei der die Abschirmung der kugelförmigen Elektrode zur Vermeidung von Bogenentladungen notwendig ist, lässt sich mit der bekannten Vorrichtung somit nicht sinnvoll durchführen.

Der vorliegenden Erfindung liegt daher die Aufgabe zugrunde, ein einfach handzuhabendes Gerät zur Behandlung einer Fläche mit einem dielektrisch behinderten Plasma der eingangs erwähnten Art anzugeben, dass ohne Weiteres auch für die Behandlung leitfähiger Oberflächen, wie beispielsweise der Hautoberfläche, geeignet ist.

Zur Lösung dieser Aufgabe ist erfindungsgemäß ein Gerät der eingangs erwähnten Art dadurch gekennzeichnet, dass das Gehäuse aus einem Griffteil und einem abnehmbaren Kopfteil zusammensetzbar ist und dass das Kopfteil einen mit der Elektrode über ein in der Elektrode eingeführtes flexibles Kabel verbundenen Anschlussstift aufweist, der im montierten Zustand von Kopfteil und Griffteil in einen passenden Führungskanal im Griffteil hineinragt und dort mit einer Hochspannungszuführung verbindbar ist.

Das erfindungsgemäße Behandlungsgerät verfügt mit der zu behandelnden Fläche nur über einen relativ geringen Kontaktbereich. Allerdings ist das Gerät aufgrund der drehbar gelagerten kugelförmigen Elektrode leicht und kontrolliert über die Fläche führbar, sodass eine Plasmabehandlung über die Fläche kontrolliert und gleichmäßig ausgeübt werden kann, indem das Gerät auf der Fläche abgerollt wird. Da die Elektrode mit der Hochspannungszuleitung innerhalb des Gehäuses des Geräts verbunden sein muss, ist die Rotationsbewegung der kugelförmigen Elektrode begrenzt. Das Überstreichen der zu behandelnden Fläche erfolgt daher mit dem erfindungsgemäßen Gerät vorzugsweise mäanderförmig oder spiralförmig.

Das erfindungsgemäße Behandlungsgerät eignet sich insbesondere für eine kosmetische Behandlung der Hautoberfläche als Vorbereitung für die anschließende Aufnahme von auf der Haut aufgetragenen hautpflegenden und -regenerierenden Substanzen. Darüber hinaus kann das erfindungsgemäße Behandlungsgerät auch für medizinische Zwecke eingesetzt werden, beispielsweise um die antimikrobielle Wirkung der Plasmabehandlung auf eine zu behandelnde Hautoberfläche auszuüben und/oder die Haut für die verbesserte Aufnahme medizinischer Wirkstoffe vorzubereiten. Weiterhin kann die Mikrozirkulation im Gewebeverbund durch das Einwirken des Plasmas positiv beeinflusst werden.

Das die kugelförmige Elektrode umgebende Dielektrikum kann eine gleichmäßige Dicke - und somit eine glatte Kontaktfläche - zur zu behandelnden Fläche - aufweisen, da in diesem Fall um die Kontaktfläche herum ein ringförmiger Bereich für die Plasmabehandlung zur Verfügung steht. Bevorzugt ist jedoch, das Dielektrikum zur zu behandelnden Fläche hin mit einer strukturierten Oberfläche zu versehen, die insbesondere durch vorstehende Noppen gebildet sein kann, wobei das Dielektrikum vorzugsweise einen Basisbereich mit gleichmäßiger Dicke aufweist, von dem aus sich die Noppen erheben. Auf diese Weise ist auch im Kontaktbereich die Ausbildung des Plasmas im Zwischenraum zwischen den Noppen gewährleistet, wodurch eine gleichmäßigere Plasmabehandlung erreicht wird. Die Noppen bilden dabei nur kleine Erhöhungen von bis zu 1 bis 2 mm und sind vorzugsweise kugelförmig ausgebildet, wobei sie dicht an dicht angeordnet sein können, sodass sich der Gasraum nicht aufgrund des Abstands der Noppen auf der Oberfläche sondern aufgrund der Kugelform der Noppen ergibt. Auf aus der Öffnung des Gehäuses herausragenden Kugelabschnittsflächen befinden sich vorzugsweise zwischen 20 und 50 Noppen, deren einhüllende, also die Maxima der Noppen verbindende Ebene, wiederum eine Kugeloberfläche bildet, die mit der Lagerausnehmung des Gehäuses korrespondiert. Demgemäß wird die Elektrode mit dem die strukturierte Oberfläche aufweisenden Dielektrikum in dem Gehäuse drehbar gehalten.

Das erfindungsgemäße Gerät kann eine extern generierte Hochspannung zugeleitet erhalten, wobei die Hochspannung in dem Gehäuse des Geräts zur kugelförmigen Elektrode geleitet wird. Bevorzugt ist jedoch, dass die Hochspannung in dem Gerät selbst generiert wird, wobei eine normale Versorgungsspannung (beispielsweise 230 V oder 110 V Netzspannung) über ein Kabel zugeführt werden kann. Dieser Geräteaufbau hat den Vorteil, dass außerhalb des Geräts keine Hochspannung geführt wird, sodass die erforderliche Gerätesicherheit wesentlich einfacher zu gewährleisten ist.

Es ist jedoch auch möglich, die Versorgungsspannung durch eine im Gerät vorhandene Batterie zu erhalten und diese in die benötigte Hochspannung umzuwandeln. Insbesondere für kosmetische Anwendungen kann diese Ausführungsform zur Erstellung eines gut handhabbaren Geräts ohne Kabelanbindung ausgenutzt werden.

Das erfindungsgemäße Gerät ist aus einem Kopfteil und einem Griffteil zusammensetzbar, wobei das Kopfteil die Elektrode mit dem Dielektrikum umfasst und die Hochspannungszuführung und ggf. der Hochspannungsgenerator und eine entsprechende Steuerung in dem Griffteil untergebracht sind. Dabei weist das Kopfteil einen mit der Elektrode verbundenen, vorzugsweise zentralen, Anschlussstift auf, der in einen passenden Führungskanal im Griffteil im montierten Zustand hineinragt und dort mit der Hochspannungszuführung verbindbar ist.

Die Verbindung zwischen dem abnehmbaren Kopfteil und dem Griffteil erfolgt vorzugsweise als Rastverbindung der Gehäuseteile. Ergänzend kann auch eine Rastverbindung zwischen dem Anschlussstift und einer den Anschlussstift kontaktierenden Anschlussbuchse vorgesehen sein.

Der isolierte Führungskanal kann gegen eine ungewollte, fahrlässige Verbindung mit der Hochspannungszuleitung dadurch geschützt werden, dass er mittels eines Querschiebers unter Federwirkung verschließbar ist, wobei der Querschieber eine Durchgangsöffnung aufweist, die mittels einer Betätigungstaste gegen die Rückstellkraft der Federwirkung in Flucht mit dem Führungskanal bringbar ist. Die Verbindung zwischen Kopfteil und Griffteil ist daher erst nach der Betätigung der Betätigungstaste möglich. Wird das Kopfteil aus dem Griffteil herausgezogen, also beispielsweise eine Rastverbindung gelöst, verschließt der Querschieber den Führungskanal aufgrund der Federwirkung, sodass die Hochspannungszuführung gegenüber dem Führungskanal abgedeckt ist.

Die Montage der kugelförmigen Elektrode gestaltet sich einfach, wenn das Gehäuse in dem die kugelförmige Elektrode lagernden Bereich im Anschluss an die Öffnung zweiteilig, insbesondere aus zwei Halbschalen, ausgebildet ist. Die kugelförmige Elektrode wird dann vor dem Verbinden der beiden Halbschalen in das kugelabschnittförmige Lager einer der Halbschalen eingelegt.

Die Erfindung soll im Folgenden anhand eines in der Zeichnung dargestellten Ausführungsbeispiels näher erläutert werden. Es zeigen:
- Figur 1: einen Schnitt in Axialrichtung durch das Gehäuse, das aus Kopfteil und Griffteil besteht, im unmontierten Zustand;
- Figur 2: den Schnitt gemäß Figur 1 für das Gehäuse im montierten Zustand;
- Figur 3: einen vergrößerten Schnitt senkrecht zu der Schnittebene aus Figur 1 durch einen Abschnitt des Kopfteils des Gehäuses;
- Figur 4: die Bestandteile des Kopfteils in explodierter Darstellung;

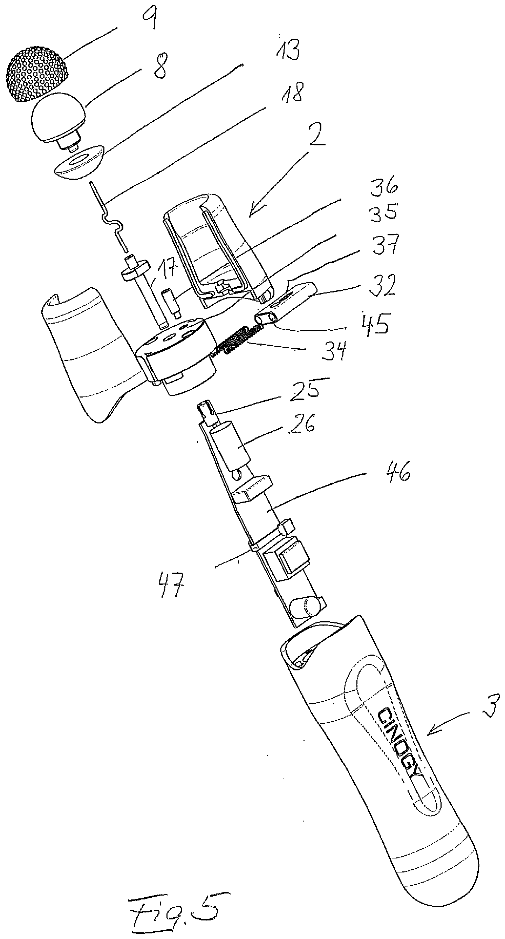

- Figur 5: die Bestandteile des vollständigen Geräts in explodierter Darstellung.

Das in der Zeichnung dargestellte Gerät weist ein Gehäuse 1 auf, das aus einem Kopfteil 2 und einem Griffteil 3 besteht. Das Kopfteil 2 besteht aus einem elastischen Kunststoffmaterial und bildet eine einstückige Wandung aus, die zu einem freien Ende 4 geringfügig konisch zuläuft. In dem freien Ende befindet sich eine stirnseitige Öffnung 5. Unmittelbar hinter der stirnseitigen Öffnung 5 bildet die Wandung des Kopfteils 2 ein kugelabschnittförmiges Lager 6 aus, in dem eine kugelförmige Elektrodenanordnung 7 in allen Richtungen drehbar gelagert ist. Die Elektrodenanordnung 7 besteht aus einer Elektrode 8 in Kugelform, die über einen überwiegenden Teil ihrer Oberfläche mit einem Dielektrikum 9 abgedeckt ist. Die Elektrodenanordnung 7 ragt mit einem kugelabschnittförmigen Teil aus der stirnseitigen Öffnung 5 heraus. Der kugelabschnittförmige Teil der Elektrodenanordnung 7 erstreckt sich über einen Kugelabschnitt, dessen Höhe weniger als 1/3, insbesondere weniger als 1/4, des Durchmessers der kugelförmigen Elektrodenanordnung 7 beträgt. Dadurch ist sichergestellt, dass die Elektrodenanordnung 7 in axialer Richtung gesehen im Bereich ihres größten Durchmessers und beidseitig davon über eine axiale Länge von wenigstens 1/5 des Durchmessers in dem kugelabschnittsförmigen Lager 6 drehbar gelagert, sodass eine sichere Lagerung gewährleistet ist. Das Dielektrikum 9 erstreckt sich über die kugelförmige Oberfläche der Elektrode 8 deutlich über den größten Durchmesser der Elek-trode 8 bezüglich des kugelabschnittsförmigen Lagers 6 hinaus und ist in einer ringförmigen Ausnehmung 10 mit einem ringförmigen Wulst 11 verankert.

Auf der von der stirnseitigen Öffnung 5 abgewandten Seite weist die kugelförmige Elektrode 8 eine ringförmige Ausnehmung auf, durch die innerhalb der Kugelkontur der Elektrode ein zylindrischer Gewindebolzen 12 gebildet wird. Auf dem axialen Ende des Gewindebolzens 12 ist ein zylindrischer Vorsprung ausgebildet, der als Anschlag 14 die Drehbewegung der kugelförmigen Elektrodenanordnung 7 begrenzt, indem der Anschlag 14 an der Innenwandung des Kopfteils anschlägt.

Der Anschlag 14 ragt in einen hohlen Innenraum 15 des Kopfteils 2, der einerseits durch die eingesetzte Elektrodenanordnung 7 und andererseits durch eine Bodenwandung 16 begrenzt ist. In der Bodenwandung 16 ist ein zylindrischer Anschlussstift 17 verankert, der zur Übertragung einer Hochspannung auf die Elektrode 8 dient. Hierzu ist die Elektrode 8 mit einem radial von dem Anschlag 14 zum Mittelpunkt verlaufenden Kanal 17' versehen, in den ein flexibles Kabel 18 eingeführt ist. Das andere Ende des Kabels 18 ist mit dem Anschlussstift 17 fest verbunden.

Das Gehäuse des Kopfteils 2 weist jenseits der Bodenwandung 16 einen umlaufenden Rand 19 mit einer ringförmigen Hinterschneidung 20 auf, mit dem eine Rastverbindung mit dem Griffteil herstellbar ist.

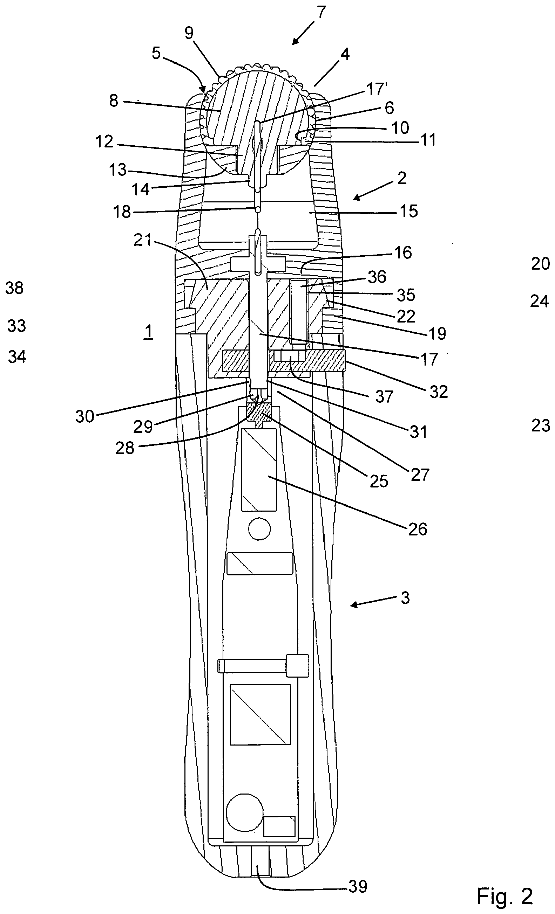

Das im Wesentlichen hohlzylindrisch und auf seiner Außenkontur ergonomisch geformte Griffteil 3 ist auf seiner zum Kopfteil 2 zeigenden Stirnseite durch einen im Wesentlichen zylindrischen Einsatz 21 aus einem isolierenden Material abgeschlossen. Der Einsatz weist an seiner Außenseite eine sägezahnförmige Erweiterung 22 auf, an die sich eine ringförmige Nut 23 anschließt. Der ringförmige Rand 19 wird zur Montage des Kopfteils 2 auf das Griffteil 3 auf die sägezahnförmige Erweiterung aufgeschoben, wodurch Rand 19 aufgedehnt wird. Beim weiteren Einschieben überwindet der Rand 19 die sägezahnförmige Erweiterung und schnappt in die Nut 23 ein, während die sägezahnförmige Erweiterung 22 in der Hinterschneidung 20 Platz findet, wie dies in Figur 2 verdeutlicht ist.

Der Einsatz 21 ist mit einem zentrischen Führungskanal 24 für den Anschlussstift 17 versehen. Am dem Kopfteil 2 abgewandten Ende des Führungskanals 24 befindet sich ein massives zylindrisches Anschlussstück 25, das mit einem Hochspannungsgenerator 26 in Form einer Hochspannungsspule verbunden ist. Das Anschlussstück 25 weist ein hohlzylindrisch ausgebildetes Aufnahmeende 27 auf, in dem sich vier zum Anschlussstift 17 hin offene Längsschlitze 28 befinden, die am massiven Bereich des Anschlussstücks 25 enden. Die Schlitze begrenzen somit vier Klemmbacken 29, die an ihrem oberen Ende mit nach innen ragenden Rastvorsprüngen 30 versehen sind. Im montierten Zustand (Figur 2) des Kopfteils 2 an dem Griffteil 3 ragt das distale Ende des Anschlussstifts 17 in das Aufnahmeende 27 des Anschlussstücks 25 hinein. Der Anschlussstift 17 weist an seinem distalen Ende eine umlaufende Rastnut 31 auf, in die die Rastwülste 30 der Klemmbacken 29 eingreifen, wenn das Kopfteil korrekt an dem Griffteil 3 montiert ist. Auf diese Weise ist eine sichere Hochspannungsverbindung ohne Gefahr von Überschlägen zwischen dem Hochspannungsgenerator 26 und der Elektrode 8 über den Anschlussstift 17 hergestellt.

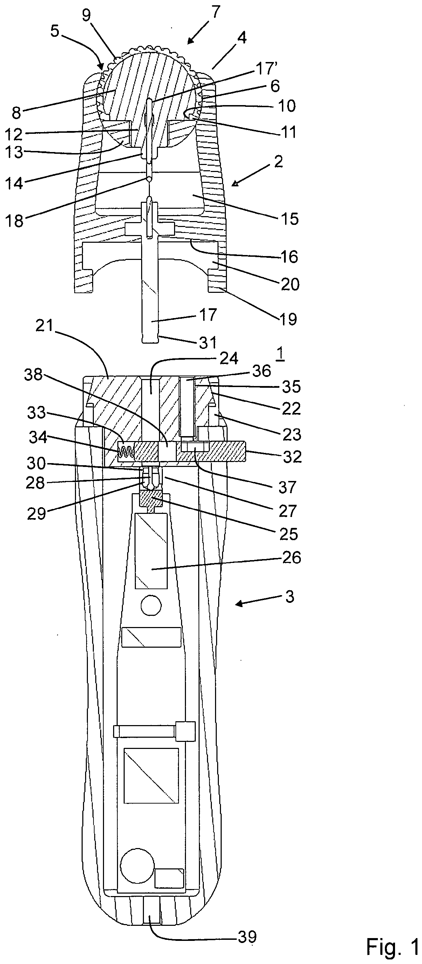

Wie Figur 1 verdeutlicht, ist der Führungskanal 24 in einer Ausgangsstellung durch einen Querschieber 32 verschlossen, der in einem Schlitz quer zur Längsrichtung des Griffteils 3 geführt ist. Der Querschieber 32 weist zwei Sacklochbohrungen 33 auf, in denen Spiralfedern 34 gelagert sind, die sich an einer stirnseitigen Schlitzwandung abstützen und im Ruhezustand den Querschieber 32 in die in Figur 1 dargestellte Ausgangsstellung drücken. Die durch die Spiralfedern 34 veranlasste Bewegung nach radial außen wird durch ein Ende eines in eine Gewindebohrung 35 eingeschraubten Bolzen 36 begrenzt, dessen Ende in eine schlitzförmige Ausnehmung 37 in der zum Kopfteil 2 zeigenden Oberfläche des Querschiebers 32 hineinragt. Der Querschieber 32 weist eine Bohrung 38 auf, die, wie Figur 2 verdeutlicht, mit dem Führungskanal 24 fluchtet, wenn der Querschieber 32, der als Betätigungstaste fungiert, gegen die Kraft der Spiralfedern 34 nach innen gedrückt wird. In diesem Zustand ist der Anschlussstift 17 in den Führungsschlitz 24 bis zum Aufnahmeende 27 des Anschlussstücks 25 einschiebbar, also das Gehäuse 1 komplett montierbar. Ist das Kopfteil 2 hingegen abgenommen, befindet sich der Querschieber 32 in der in Figur 1 dargestellten Position, in der er den Führungskanal 24 zum Anschlussstück 25 des Hochspannungsgenerators 26 hin verschließt und so eine Sicherungsfunktion gegen das Berühren der Hochspannung bewirkt.

Die Figuren 1 und 2 lassen erkennen, dass im Griffteil 3 weitere Bauelemente einer elektronischen Schaltung und Steuerung untergebracht sind. Das verschlossene, dem Einsatz 21 gegenüberliegende Ende des Gehäuses 1 weist eine Durchgangsöffnung 39 zur Durchführung eines Anschlusskabeis auf. Vorzugsweise wird über das Anschlusskabel die im Griffteil 3 befindliche Schaltung mit einer üblichen Netzspannung (230 V oder 110 V Wechselspannung) mit einer anderen zur Verfügung stehenden Versorgungsspannung, die auch eine Gleichspannung sein kann, versorgt. Grundsätzlich ist es auch denkbar, über die Durchgangsöffnung 39 bereits eine Hochspannung zuzuführen, wodurch der Hochspannungsgenerator 26 in dem Griffteil 3 entfallen könnte. Bevorzugt ist jedoch die Zuführung einer üblichen Netzspannung und die Erzeugung der für die Plasmabehandlung benötigten Hochspannungen durch den Hochspannungsgenerator 26 in dem Gehäuse 1.

Sofern das Griffteil 3 mit einer eigenen Stromversorgung in Form einer Batterie oder eines aufladbaren Akkumulators erfolgt, bleibt die Durchgangsöffnung 39 unbenutzt und kann ggf. verschlossen werden.

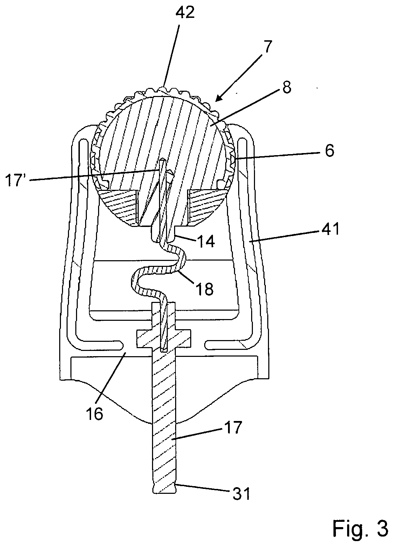

Die vergrößerte Darstellung der Figur 3 zeigt, dass das zwischen dem Anschlussstift 17 und der Elektrode 8 angeordnete Kabel 18 mit einer Überlänge zu einer Schlaufe gelegt ist, damit die Elektrodenanordnung 7 drehbar ist und das Kabel 18 dieser Drehbewegung folgen kann. Die Überlänge ist so groß ausgebildet, dass das Kabel innerhalb des durch den Anschlag 14 begrenzten Bereichs die Rotation der Elektrodenanordnung 7 mitmachen kann, um gespannt zu werden.

Figur 3 lässt ferner erkennen, dass das durch die Bohrung 17' eingeführte Kabelende durch eine Madenschraube festlegbar ist, die in eine Gewindebohrung 40, die in einem spitzen Winkel zur Bohrung 17' verläuft und die Bohrung 17' kreuzt, einschraubbar ist.

Ferner ist in Figur 3 erkennbar, dass die Wandung des Kopfteils Aufnahmenuten 41 aufweist, die in einer Halbschale des Kopfteils angeordnet sind, wie unten näher erläutert wird.

Die vergrößerte Darstellung der Figur 3 verdeutlicht die Ausbildung der strukturierten Oberfläche des Dielektrikums 9 durch vorstehende, etwa kugelförmig ausgebildete Noppen 42, die praktisch dicht an dicht angeordnet sind und allenfalls einen Abstand aufweisen, der weniger als die Hälfte des Noppendurchmessers beträgt.

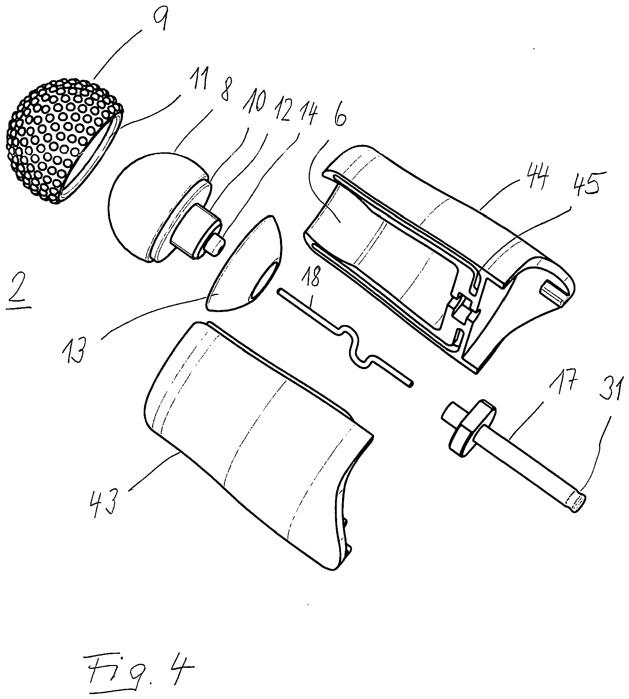

Die explodierte Darstellung der Bestandteile des Kopfteils 2 in Figur 4 verdeutlicht die kugelförmige Elektrode 8, die nur insoweit kugelförmig ist, wie sie in dem kugelabschnittförmigen Lager 6 drehbar gelagert ist. Außerhalb dieses Bereichs befindet sich die ringförmige Ausnehmung 10, der Gewindebolzen 12 und der Anschlag 14.

Das Dielektrikum 9 wird auf den kugelförmigen Teil der Elektrode 8 aufgebracht, beispielsweise durch Aufschrumpfen. Dabei greift der ringförmige Wulst 11 in die ringförmige Ausnehmung 10 der Elektrode 8 ein. Die so hergestellte Verbindung zwischen Elektrode 8 und Dielektrikum 9 wird durch das Verschlussteil 13, das auf den Gewindebolzen 12 aufgeschraubt wird, gesichert.

Das Dielektrikum 9 kann auch durch einen Beschichtungsvorgang mit der Elektrode 8 verbunden sein.

Das Gehäuse des Kopfteils 2 besteht aus zwei Halbschalen 43, 44, die spiegelsymmetrisch ausgebildet sind, wobei jedoch die Halbschale 43 die Aufnahmenuten 41 aufweist, während die Halbschale 44 mit einer entsprechend geformten Steganordnung 45 versehen ist, die in die Aufnahmenuten eingedrückt werden, wenn die beiden Halbschalen 43, 44 zu dem Gehäuse des Kopfteils 2 gegeneinander gedrückt werden. Die Verbindung der beiden Halbschalen 43, 44 kann beispielsweise durch eine Rasteinrichtung oder durch eine Verklebung sichergestellt werden.

Die explodierte Darstellung des Gesamtgeräts zeigt Aufnahmekammern 45' für die beiden Spiralfedern 34 in dem Querschieber 32. Ferner wird deutlich, dass sich die gesamte Elektronik einschließlich des Hochspannungsgenerators 36 und des Anschlussstücks 25 auf einer Platine 46 befindet, die im Griffteil 3 des Gehäuses 1 gelagert und beispielsweise mittels eines Spannstreifens 47 gegen Verschiebungen gesichert ist.

Das abnehmbare Kopfteil 2 ermöglicht eine hygienische Verwendung des Geräts, beispielsweise in einem Kosmetikstudio, bei mehreren Personen nacheinander, indem jeweils das Kopfteil ausgewechselt wird, sodass das benutzte Kopfteil einer Reinigung bzw. Sterilisation unterzogen werden kann.

Für bevorzugte Ausführungsformen des erfindungsgemäßen Geräts liegt der Durchmesser der Elektrodenanordnung 7 zwischen 10 und 50 mm. Die Höhe der Noppen ist ≤ 2 mm. Die Dicke des Dielektrikums 9 (ohne Noppen) liegt vorzugsweise zwischen 0,1 und 1 mm.

## Patentansprüche

1. Gerät zur Behandlung einer Fläche mit einem dielektrisch behinderten Plasma, wobei die Fläche als Gegenelektrode fungiert, mit einem Gehäuse (1), in dem sich eine Hochspannungszuleitung, eine mit der Hochspannungszuleitung verbundene Elektrode (8) und ein die Elektrode (8) gegenüber der Fläche abschirmendes Dielektrikum (9) befindet, wobei die Elektrode (8) die Form einer Kugel aufweist, die zumindest begrenzt in dem Gehäuse (1) drehbar gelagert ist und mit einem Kugelabschnitt aus einer stirnseitigen Öffnung (5) des Gehäuses (1) herausragt und die Elektrode (8) so mit dem Dielektrikum (9) verbunden ist, dass ihr aus dem Gehäuse (1) herausragender Kugelabschnitt in jeder möglichen Drehstellung durch das Dielektrikum (9) abgedeckt ist, **dadurch gekennzeichnet, dass** das Gehäuse (1) aus einem Griffteil (3) und einem abnehmbaren Kopfteil (2) zusammensetzbar ist und dass das Kopfteil (2) einen mit der Elektrode (8) über ein in der Elektrode (8) eingeführtes flexibles Kabel (18) verbundenen Anschlussstift (17) aufweist, der im montierten Zustand von Kopfteil (2) und Griffteil (3) in einen passenden Führungskanal (24) im Griffteil (3) hineinragt und dort mit einer Hochspannungszuführung verbindbar ist.

2. Gerät nach Anspruch 1, **dadurch gekennzeichnet, dass** das Dielektrikum (9) zur zu behandelnden Fläche hin eine strukturierte Oberfläche aufweist.

3. Gerät nach Anspruch 2, **dadurch gekennzeichnet, dass** die strukturierte Oberfläche vorstehende Noppen (42) aufweist.

4. Gerät nach einem der Ansprüche 1 bis 3, **dadurch gekennzeichnet, dass** der Führungskanal (24) durch einen Querschieber (32) unter Federwirkung verschließbar ist und dass der Querschieber (32) eine Durchgangsöffnung (39) aufweist, die mittels einer Betätigungstaste gegen die Rückstellkraft der Federwirkung in Flucht mit dem Führungskanal (24) bringbar ist.

## Claims

1. A device for treating a surface with a dielectric barrier plasma, wherein the surface functions as counter electrode, comprising a housing (1), in which a high-voltage feed line, an electrode (8) connected to the high-voltage feed line, and a dielectric (9) which screens the electrode (8) with respect to the surface are located, in which the electrode (8) has the shape of a sphere which is rotatably mounted at least to a limited extent in the housing (1) and protrudes via a spherical portion from an end-face opening (5) in the housing (1), and the electrode (8) is connected to the dielectric (9) such that the spherical portion of said electrode protruding from the housing (1) is covered by the dielectric (9) in every possible rotational position of the electrode, **characterized in that** the housing (1) is composed of a handle part (3) and a removable head part (2) and that the head part (2) comprises a connecting pin (17) being connected to a flexible cable (18) which is introduced in the electrode (8), which connecting pin, when the head part (2) and the handle part (3) are mounted together, projects into a complementary guide channel (24) in the handle part (3) and can there be connected to the high-voltage supply.

2. The device as claimed in claim 1, **characterized in that** the dielectric (9) has a structured surface relative to the surface to be treated.

3. The device as claimed in claim 2, **characterized in that** the structured surface has protruding nubs (42).

4. The device as claimed in claim 4, **characterized in that** the guide channel (24) can be closed by a transverse slide (32) under spring effect, and **in that** the transverse slide (32) has a through-opening (39), which can be brought into alignment with the guide channel (24) by means of an actuation button, against the restoring force of the spring effect.

## Revendications

1. Appareil de traitement d'une surface par un plasma empêché par voie diélectrique, la surface faisant office d'électrode antagoniste, comportant un boîtier (1) dans lequel se trouve une ligne d'alimentation haute tension, une électrode (8) connectée à la ligne haute tension et un diélectrique (9) blindant l'électrode (8) par rapport à la surface, l'électrode (9) présente la forme d'une sphère qui est montée rotative au moins de façon limitée dans le boîtier (1) et qui dépasse par une partie de sphère hors d'une ouverture (5) côté frontal du boîtier (1), et l'électrode (8) est reliée au diélectrique (9) de telle sorte que sa partie de sphère dépassant hors du boîtier (1) est recouverte par le diélectrique (9) dans chaque position de rotation possible, **caractérisé en ce que** le boîtier (1) est susceptible d'être constitué par une partie de poignée (3) et par une partie de tête amovible (2) et **en ce que** la partie de tête (2) comprend une tige de raccordement (17) reliée à l'électrode (8) par un câble flexible (18) introduit dans l'électrode (8), tige qui, dans l'état monté de la partie de tête (2) et de la partie poignée (3) dépasse dans un canal de guidage adapté (24) dans la partie de tête et qui est susceptible d'être connectée ici à une ligne d'alimentation haute tension.

2. Appareil selon la revendication 1, **caractérisé en ce que** le diélectrique (9) présente une surface structurée vers la surface à traiter.

3. Appareil selon la revendication 1, **caractérisé en ce que** la surface structurée présente des boutons saillants (42).

4. Appareil selon l'une des revendications 1 à 3, **caractérisé en ce que** le canal de guidage (24) est susceptible d'être refermé par un poussoir transversal (32) sous l'effet d'un ressort et **en ce que** le poussoir transversal (32) présente une ouverture traversante (39) qui est susceptible d'être amenée en alignement avec le canal de guidage (24) au moyen d'une touche d'actionnement à l'encontre de la force de rappel du ressort.
